# EUROPEAN PATENT APPLICATION

(11) **EP 4 335 453 A1**
(43) Date of publication of application: **13.03.2024**
(21) Application number: 22806517.3
(22) Date of filing: 28.04.2022
(51) Int. Cl.: A61K 38/17, A61P 3/06, A61P 25/00, A61P 25/16, A61P 25/28

(54) **PHARMACEUTICAL USE OF HYPOXIA-INDUCIBLE LIPID DROPLET-ASSOCIATED PROTEIN OR TRUNCATED FRAGMENT THEREOF**

(30) Priority: 08.05.2021 CN 202110514625
(71) Applicant: Westlake University, Hangzhou, Zhejiang 310024 (CN)
(72) Inventor: ZOU, Yilong, Hangzhou, Zhejiang 310024 (CN); SHI, Taotao, Hangzhou, Zhejiang 310024 (CN); WU, Tianshu, Hangzhou, Zhejiang 310024 (CN); WANG, Fengxiang, Hangzhou, Zhejiang 310024 (CN); LU, Yang, Hangzhou, Zhejiang 310024 (CN); PAN, Zijian, Hangzhou, Zhejiang 310024 (CN); HAN, Jingrong, Hangzhou, Zhejiang 310024 (CN); PENG, Qin, Hangzhou, Zhejiang 310024 (CN); WANG, Yixin, Hangzhou, Zhejiang 310024 (CN)
(74) Representative: Meissner Bolte Partnerschaft mbB
(86) International application number: PCT/CN2022/089740
(87) International publication number: WO 2022/237548

(57) **Abstract**

The present invention relates to the field of biomedicine, and relates to a pharmaceutical use of a hypoxia-inducible lipid droplet-associated protein or a truncated fragment thereof. Specifically, the present invention relates to a use of a hypoxia-inducible lipid droplet-associated protein or a truncated active fragment thereof in the preparation of a drug for treating or preventing hyperlipidemia, hyperlipidemia complications or neurodegenerative diseases, or a use of the hypoxia-inducible lipid droplet-associated protein or the truncated active fragment thereof in the preparation of a drug for reducing the apolipoprotein level, low-density lipoprotein level, or very low-density lipoprotein level in blood or cerebrospinal fluid.

## Description

### Technical Field

The present invention belongs to the field of biomedicine, and relates to a pharmaceutical use of hypoxia-inducible lipid droplet-associated protein or a truncated fragment thereof. Specifically, the present invention relates to a use of hypoxia-inducible lipid droplet-associated protein or a truncated active fragment thereof in the preparation of a medicament for the treatment or prevention of hyperlipidemia, a hyperlipidemia complication or a neurodegenerative disease, or in the preparation of a medicament for reducing the level of apolipoprotein or the level of low-density lipoprotein in blood or in cerebrospinal fluid.

### Background Art

Hyperlipidemia is a very common metabolic disease in modern society. Its main clinical manifestations are abnormal increase in the content of cholesterol, triglyceride and apolipoprotein in the patient's blood. Epidemiological investigations have revealed a high incidence of hyperlipidemia, with over 13% of males and more than 23% of females exhibiting hyperlipidemic conditions among people aged over 60 years in my country. Persistent hyperlipidemia is known to promote atherosclerosis, leading to various cardiovascular complications, hypertension, hyperglycemia, hyperuricemia, and other associated disorders.

In clinical practice, statins are the most used lipid-lowering drugs. They primarily function by inhibiting key enzymes involved in cellular cholesterol synthesis, thereby reducing cholesterol release into the bloodstream, effectively lowering lipid levels. Despite the wide use, statins exhibit notable limitations, including limited efficacy in reducing triglycerides and lipoproteins, as well as the propensity to induce adverse effects such as liver damage and myalgia. Therefore, there is a significant clinical need to explore lipid-lowering drugs with specific effects on triglycerides and lipoproteins.

Central nervous system (CNS) diseases are common diseases that threaten human health. The central nervous system is rich in lipids, and lipid metabolism plays an important role in maintaining the normal physiological functions of the nervous system. Many central nervous system diseases are closely related to aberrant lipid metabolism. Studies have shown that lipid metabolism is significantly altered in some neurodegenerative diseases, such as multiple sclerosis, Alzheimer's disease and Parkinson's disease.

Hypoxia-inducible lipid droplet-associated protein (HILPDA) is a target protein (encoded by *HILPDA* gene) regulated by hypoxia-inducible factor 1/2 (HIF1/2) or peroxisome proliferator-activated receptor (PPAR), which is mainly located in lipid droplets (lipid droplets) in cells, and promotes lipid accumulation by inhibiting the activity of triglyceride hydrolase (ATGL) in lipid droplets. HILPDA consists of 63 amino acids in humans and 64 amino acids in mice.

Previously, there was a small amount of evidence that HILPDA, as a small secreted protein, was highly expressed in the blood of patients with clear cell renal cell carcinoma (Cancer Res. 2005 Jun 1;65(11):4817-26.), but this conclusion was subsequently rejected by another research team (FASEB J. 2010 Nov;24(11):4443-58.), so whether it is a secreted protein is still controversial. The present inventors have found through research that HILPDA does have secretory characteristics and is widely present in various body fluids including blood and cerebrospinal fluid.

Apolipoproteins are amphipathic proteins that can bind lipids (e.g., fat and cholesterol), which are mainly divided into several major categories, including apolipoprotein A, apolipoprotein B, apolipoprotein C, apolipoprotein D, apolipoprotein E, apolipoprotein F, apolipoprotein H, apolipoprotein L, apolipoprotein M, apolipoprotein (a), of which apolipoprotein A (APOA) is composed of four subtypes: APOA1, APOA2, APOA4 and APOA5. Apolipoprotein B (APOB) can be divided into two subtypes, APOB-48 (produced mainly in the small intestine) and APOB-100 (produced mainly in the liver). In addition, apolipoprotein C (APOC) can also be divided into four subtypes: APOC1, APOC2, APOC3, and APOC4. Apolipoproteins wrap and surround insoluble lipids in complex and diverse combinations to form water-soluble lipoproteins (also known as lipoprotein particles, for example, lipoproteins distributed in the blood system are mainly divided into chylomicrons (CM), very-low-density lipoprotein (VLDL), intermediate-density lipoprotein (IDL), low-density lipoprotein (LDL), high-density lipoprotein (HDL), etc.) that are responsible for the transport of lipids in the aqueous circulatory system (e.g., blood and cerebrospinal fluid, etc.). In addition to assembling lipoprotein particles and increasing the solubility of lipids, apolipoproteins are also capable of directly interacting with lipoprotein receptors distributed in specific tissues or organs (e.g., liver, adrenal gland, or some cells in the brain, etc.), triggering lipoprotein receptor-mediated endocytosis, mediating the uptake and clearance of lipoproteins, thereby maintaining lipid (or lipoprotein) metabolic homeostasis in the circulatory system.

Dysfunction of apolipoproteins, or the lipoproteins they give rise to, has been closely linked to a multitude of diseases and metabolic disorders, with a notable emphasis on cardiovascular disorders, specifically hyperlipidemia and atherosclerosis stemming from prolonged hyperlipidemia, as well as neurodegenerative conditions such as Alzheimer's disease. To illustrate, elevated levels of APOC3 in the bloodstream hinder the enzymatic activity of lipoprotein lipases situated on the endothelial cells of capillaries, resulting in the accumulation of triglyceride-rich lipoproteins such as CM, VLDL and IDL, thereby precipitating hypertriglyceridemia. Similarly, the significant accumulation of lipoproteins enriched with APOB, including CM, VLDL, IDL and LDL, within the bloodstream can precipitate hyperlipidemia and give rise to severe cardiovascular conditions, notably atherosclerosis. Moreover, genetic polymorphisms within the APOE gene, particularly the APOE2 variant, demonstrate a robust association with hyperlipidemia and its attendant complications.

In the human body, different types of apolipoproteins participate in the formation of distinct lipoprotein particles. For example, APOE exists in three different forms: APOE2, APOE3, and APOE4. APOE2 exhibits markedly reduced affinity for lipoprotein receptors, hindering the clearance of lipoproteins such as CM, VLDL, and IDL, leading to the excessive accumulation of these lipoproteins and the development of Type III hyperlipidemia.

APOE4 is considered to be one of the strongest genetic risk factors for the development of late-onset Alzheimer's disease. Although the molecular mechanism is still unclear, one possible reason is that compared with APOE3 or APOE2, the APOE4 in brain interstitial fluid (or cerebrospinal fluid) is more likely to form insoluble amyloid plaques with amyloid Aβ, resulting in blocked neuronal information exchange and causing Alzheimer's disease.

Furthermore, related research has shown that the deletion of HILPDA in mice models lacking the apolipoprotein APOE can reduce the formation of foam cells, a characteristic cell type derived from macrophages that accumulate excessive lipids, thus mitigating the progression of atherosclerosis. These findings unveil the role of HILPDA in promoting the development of atherosclerosis in specific APOE-deficient mouse models. However, due to the employment of a genetic knockout approach in this study, specific mechanisms governing HII,PDA's involvement in the regulation of atherosclerosis progression were still not elucidated.

To date, there have been no reports of HILPDA lowering lipoprotein and lipid levels in the bloodstream or cerebrospinal fluid.

### Contents of the Invention

Through extensive research and innovative efforts, the present inventors have surprisingly found that hypoxia-inducible lipid droplet-associated protein (also known as lipid-lowering peptide in the present invention, and named as Liplorin in English) can effectively promote the clearance of apolipoprotein (including but not limited to apolipoprotein E, which is named as Apolipoprotein E or ApoE) and lipids in the blood by various cells cultured *in vitro,* thereby reducing the levels of apolipoproteins (including but not limited to ApoE) and lipoproteins in the blood. In addition, the present inventors found through research that a truncated sequence of HILPDA can effectively promote the decrease of the content of apolipoprotein E (ApoE) in the cerebrospinal fluid circulation system of rats. The following inventions are thus provided:

One aspect of the present invention relates to a use of a hypoxia-inducible lipid droplet-associated protein or a truncated active fragment thereof in the preparation of a medicament for the treatment or prevention of hyperlipidemia, a hyperlipidemia complication or a neurodegenerative disease, or in the preparation of a medicament for lowering apolipoprotein level, low-density lipoprotein level or very-low-density lipoprotein level in cerebrospinal fluid.

In some embodiments of the present invention, in the use, the hypoxia-inducible lipid droplet-associated protein has an amino acid sequence as set forth in any one of SEQ ID NOs: 1 to 12.

In the present invention, unless otherwise specified, the term "truncated active fragment thereof" refers to a truncated fragment of hypoxia-inducible lipid droplet-associated protein, which has the same or similar biological activity or pharmacological activity as the hypoxia-inducible lipid droplet-associated protein.

In some embodiments of the present invention, in the use, the truncated active fragment of the hypoxia-inducible lipid droplet-associated protein has an amino acid sequence as set forth in any one of SEQ ID NOs: 13 to 15.

In some embodiments of the present invention, in the use, the hyperlipidemia is a type III hyperlipidemia.

In some embodiments of the present invention, in the use, the hyperlipidemia complication is selected from the group consisting of atherosclerosis, coronary heart disease, hypertension, hyperglycemia and hyperuricemia; preferably atherosclerosis, coronary heart disease, hypertension, hyperglycemia or hyperuricemia caused by long-term (e.g., more than 1 year or more than 3 years) hyperlipidemia.

In some embodiments of the present invention, in the use, the neurodegenerative disease is selected from the group consisting of multiple sclerosis, Alzheimer's disease and Parkinson's disease.

In some embodiments of the present invention, in the use, the apolipoprotein is one or more selected from the group consisting of human APOE2, APOE3 and APOE4.

Another aspect of the present invention relates to a hypoxia-inducible lipid droplet-associated protein or a truncated active fragment thereof, which is used for the treatment or prevention of hyperlipidemia, a hyperlipidemia complication or a neurodegenerative disease, or used for reducing apolipoprotein level, low-density lipoprotein level, or very-low-density lipoprotein level in blood or cerebrospinal fluid.

In some embodiments of the present invention, the hypoxia-inducible lipid droplet-associated protein or a truncated active fragment thereof, wherein, the hypoxia-inducible lipid droplet-associated protein has an amino acid sequence as set forth in any one of SEQ ID NOs: 1 to 12.

In some embodiments of the present invention, the hypoxia-inducible lipid droplet-associated protein or a truncated active fragment thereof, wherein, the truncated active fragment of the hypoxia-inducible lipid droplet-associated protein has an amino acid sequence as set forth in SEQ ID NOs: 13 to 15.

In some embodiments of the present invention, the hypoxia-inducible lipid droplet-associated protein or a truncated active fragment thereof, wherein the hyperlipidemia is a type III hyperlipidemia.

In some embodiments of the present invention, the hypoxia-inducible lipid droplet-associated protein or a truncated active fragment thereof, wherein the hyperlipidemia complication is selected from the group consisting of atherosclerosis, coronary heart disease, hypertension, hyperglycemia and hyperuricemia; preferably atherosclerosis, coronary heart disease, hypertension, hyperglycemia or hyperuricemia caused by long-term (e.g., more than 1 year or more than 3 years) hyperlipidemia.

In some embodiments of the present invention, the hypoxia-inducible lipid droplet-associated protein or a truncated active fragment thereof, wherein the neurodegenerative disease is selected from the group consisting of multiple sclerosis, Alzheimer's disease and Parkinson's disease.

In some embodiments of the present invention, the hypoxia-inducible lipid droplet-associated protein or a truncated active fragment thereof, wherein the apolipoprotein is one or more selected from the group consisting of human APOE2, APOE3 and APOE4.

Yet another aspect of the invention relates to a method of treating or preventing hyperlipidemia, a hyperlipidemia complication, or a neurodegenerative disease, or a method of reducing apolipoprotein level, low-density lipoprotein level, or very-low-density lipoprotein level in blood or cerebrospinal fluid, which comprises a step of administering an effective amount of hypoxia-inducible lipid droplet-associated protein or a truncated active fragment thereof to a subject in need thereof.

In some embodiments of the present invention, in the method, the hypoxia-inducible lipid droplet-associated protein has an amino acid sequence as set forth in any one of SEQ ID NOs: 1 to 12.

In some embodiments of the present invention, in the method, the truncated active fragment of the hypoxia-inducible lipid droplet-associated protein has an amino acid sequence as set forth in any one of SEQ ID NOs: 13 to 15.

In some embodiments of the present invention, in the method, the hyperlipidemia is a type III hyperlipidemia.

In some embodiments of the present invention, in the method, the hyperlipidemia complication is selected from the group consisting of atherosclerosis, coronary heart disease, hypertension, hyperglycemia and hyperuricemia; preferably atherosclerosis, coronary heart disease, hypertension, hyperglycemia or hyperuricemia caused by long-term (e.g., more than 1 year or more than 3 years) hyperlipidemia.

In some embodiments of the present invention, in the method, the neurodegenerative disease is selected from the group consisting of multiple sclerosis, Alzheimer's disease and Parkinson's disease.

In some embodiments of the present invention, in the method, the apolipoprotein is one or more selected from the group consisting of human APOE2, APOE3 and APOE4.

In some embodiments of the present invention, in the method, the subject is a mammal, such as a human, a gorilla, a chimpanzee, a monkey, a rat, a mouse, a pig, a cow, a horse or a sheep, preferably a human.

The present invention also relates to a truncated active fragment of hypoxia-inducible lipid droplet-associated protein, which has an amino acid sequence as set forth in any one of SEQ ID NOs: 13 to 15.

The present invention also relates to a pharmaceutical composition, which comprises an effective amount of a hypoxia-inducible lipid droplet-associated protein, or an effective amount of a truncated active fragment of the hypoxia-inducible lipid droplet-associated protein of the present invention, and one or more pharmaceutically acceptable excipients.

In some embodiments of the present invention, in the pharmaceutical composition, the hypoxia-inducible lipid droplet-associated protein has an amino acid sequence as set forth in any one of SEQ ID NOs: 1 to 12.

In some embodiments of the present invention, in the pharmaceutical composition, the truncated active fragment of the hypoxia-inducible lipid droplet-associated protein has an amino acid sequence as set forth in any one of SEQ ID NOs: 13 to 15.

In some embodiments of the present invention, the pharmaceutical composition comprises an effective amount of hypoxia-inducible lipid droplet-associated protein, or an effective amount of a truncated active fragment of the hypoxia-inducible lipid droplet-associated protein of the present invention, and one or more pharmaceutically acceptable excipients.

In the pharmaceutical composition of the present invention, the hypoxia-inducible lipid droplet-associated protein or a truncated active fragment thereof is used as the main drug (medicinal ingredient).

In some embodiments of the invention, the pharmaceutical composition described above further comprises one or more blood lipid-lowering drugs such as statins.

Yet another aspect of the present invention relates to a combination of pharmaceutical products, which comprises a first product and a second product in separate packages,
wherein,
the first product comprises the pharmaceutical composition according to any one of the items of the present invention;
the second product comprises one or more lipid-lowering drugs such as statins;
optionally, which further comprises a product manual.

In the present invention, the "first" or "second" in the "first product" or "second product" is only for distinguishing or clearly denoting, and has no order meaning.

In the present invention, the statins are already marketed statins, including but not limited to: lovastatin, simvastatin, pravastatin, fluvastatin, atorvastatin, rosuvastatin or pitavastatin.

The present invention also comprises items 1 to 9 related to the following:
1. Use of hypoxia-inducible lipid droplet-associated protein (HILPDA) in the preparation of a medicament for reducing a content of apolipoproteins and lipids in the circulatory system, such as the blood circulatory system.
2. Use of hypoxia-inducible lipid droplet-associated protein (HILPDA) in the preparation of a medicament for preventing or treating a hyperlipidemia-related disease.
3. Use of HILPDA in the preparation of a medicament for preventing or treating a neurodegenerative disease.
4. The use according to item 1 or 2, wherein the hyperlipidemia-related disease is selected from the group consisting of hyperlipidemia and complication thereof.
5. The use according to item 4, wherein the complication includes atherosclerosis caused by long-term hyperlipidemia, and cardiovascular disease, hypertension, hyperglycemia, and hyperuricemia caused by long-term hyperlipidemia.
6. The use according to item 1 or 3, wherein the neurodegenerative disease includes multiple sclerosis, Alzheimer's disease, and Parkinson's disease.
7. The use according to any one of items 1 to 3, wherein the hypoxia-inducible lipid droplet-associated protein has an amino acid sequence as set forth in any one selected from the group consisting of SEQ ID NOs: 1 to 15.
8. The use according to any one of items 1 to 3, wherein the hypoxia-inducible lipid droplet-associated protein is further modified.
9. The use according to any one of items 1 to 3, wherein the medicament is used to treat a human or other mammal.

The present invention also relates to the following aspects:
In one aspect, the present invention provides a use of hypoxia-inducible lipid droplet-associated protein (HILPDA) in the preparation of a medicament for reducing the level of apolipoprotein and lipid in blood.

In another aspect, the present invention provides a use of hypoxia-inducible lipid droplet-associated protein (HILPDA) in the preparation of a medicament for preventing or treating a hyperlipidemia-related disease.

The present invention also provides a use of HILPDA in the preparation of a medicament for preventing or treating a neurodegenerative disease related to lipid metabolism.

In one embodiment, the HILPDA can be an HILPDA from a human (Homo sapiens), a chimpanzee (Pan troglodytes) or a gorilla (Gorilla), which has an amino acid sequence as set forth in SEQ ID NO: 1 (MKHVLNLYLLGVVLTLLSIFVRVMESLEGLLESPSPGTSWTTRSQLANTEPTKGLPDHP SRSM).

In another embodiment, the HILPDA may be a mouse (Mus musculus) HILPDA, which has an amino acid sequence as set forth in SEQ ID NO: 2 (MKFMLNLYVGIMLTLL SIF VRVMESLGGLLESPLPGS S WITRGQLANTQPPKGLPDHPS RGVQ).

In another embodiment, the HILPDA may be an HILPDA from a green monkey (Chlorocebus sabaeus), which has an amino acid sequence as set forth in SEQ ID NO: 3 (MKHMLNLYLLGVVLTLLSIFVRVMESLEGLLENPSPGTSWTTRSQLANTEPTKGLPDHP SRSI).

In another embodiment, the HILPDA may be an HILPDA from a Qinling golden monkey (Rhinopithecus roxellana), which has an amino acid sequence as set forth in SEQ ID NO: 4 (MKHVLNLYLLGVVLTLLSIFVRVMESLEGLLENPLPGTSWTTRSQLTNTEPTKGLPDHP SRSM).

In another embodiment, the HILPDA may be an HILPDA from a Macaca fascicularis, which has an amino acid sequence as set forth in SEQ ID NO: 5 (MKHMLNLYLLGVVLTLLSIFVRVMESLEGLLENPSPGTSWTTRSQLANTEPTKGLPDHP SRSM).

In another embodiment, the HILPDA may be an HILPDA from a domestic pig (Sus scrofa), which has an amino acid sequence as set forth in SEQ ID NO: 6 (MKQVLQLYLLGVVLTLLSVFVRLMETLGGLLESPSPGSFWTTRGQLANTEAKSLPEHPS RGV).

In another embodiment, the HILPDA may be an HILPDA from a dog (Canis familiaris), which has an amino acid sequence as set forth in SEQ ID NO: 7 (MKPMLNLYLLGVVLTLLSIFVRLMESLGGILESPLLGSSWTTRGHTASAEPPRAFQTIHP ERCDKTSLHQL).

In another embodiment, the HILPDA may be an HILPDA from a goat (Capra hircus), which has an amino acid sequence as set forth in SEQ ID NO: 8 (MKHVLNLYLLGGVLTLLSIFVRLMESLEGLLESPPSPGSSWATRGQLANTEPPRAFQTIH PEGCDKTSLHRI,).

In another embodiment, the HILPDA may be an HILPDA from a horse (Equus caballus), which has an amino acid sequence as set forth in SEQ ID NO: 9 (MKHILNLYLLGVVLTLLSIFVRLMESLEGLLESPSPGSSWTTRSQLVSTRPPRAFQTIHPE GCDKTSVHRL).

In another embodiment, the HILPDA may be an HILPDA from a bovine (Bos taurus), which has an amino acid sequence as set forth in SEQ ID NO: 10 (MKHMLNLYLLGGVMTLLSIFIGLMESLEACWRACLLGAGPGPEVSLPTQSPQGSSRPSI QRGVIRPP STVYNLGTV SKIPPTLPVPGQAS STS VLSEN).

In another embodiment, the HILPDA may be an HILPDA from an Oryctolagus cuniculus, which has an amino acid sequence as set forth in SEQ ID NO: 11 (MKHVLNLYVLGVVLALLSIFVRVMESLESLVESPSAGNSWSTRGQLAGAEPPKGLPDP PSRGMR).

In another embodiment, the HILPDA may be a corresponding protein from a rat (Rattus norvegicus), which has an amino acid sequence as set forth in SEQ ID NO: 12 (MKYMLNLYVLGVMLTLLSIFVRLMESLGGLLENPLPGSSWITRGHLANTQPPRGLPDH PSRGVQ).

In one embodiment, the medicament is used for the treatment of a human or other mammal. The other mammal refers to a mammal other than human, such as ape, monkey, pig, dog, cow, sheep, horse, donkey, rabbit, and the like.

In some embodiments, the HILPDA from human can be used to treat a human or other mammal.

In some embodiments, the HILPDA from other mammal can be preferably used for the treatment of a domesticated pet of closely related species (e.g., without reproductive isolation) of the corresponding mammal. For example, that from dog can be used to treat a dog or wolf.

In the present invention, preferably, one or more amino acids among the amino acids constituting HILPDA may be D-form amino acids.

In the present invention, preferably, the HILPDA can be further modified.

In the present invention, the HILPDA can be modified by various technical means known in the art, as long as the modification does not significantly deteriorate the function of HILPDA to effectively promote the absorption of apolipoprotein and lipoprotein in serum. There are many types of polypeptide modifications, and according to different modification sites, they can be divided into truncation, N-terminal modification, C-terminal modification, side chain modification, amino acid modification, backbone modification, L-type amino acid conversion to D-type amino acid, etc. Peptide modification can effectively alter the physical and chemical properties of peptide compounds, increase water solubility, prolong the action time in vivo, alter their biodistribution, reduce immunogenicity, and decrease toxic or side effects.

For example, the modification methods include, but are not limited to, truncation of polypeptide, modification by connecting stearic acid to terminal (e.g., N-terminal), amination modification, acetylation modification, biotinylation modification, fluorescent labeling modification, polyethylene glycol modification, prenylation modification, myristoylation and palmitoylation modification, phosphorylation modification, glycosylation modification, polypeptide conjugate modification, special amino acid modification, etc.

Particularly, in one embodiment, the truncated sequence of HILPDA may be a truncated product of the corresponding protein derived from a human (Homo sapiens), a chimpanzee (Pan troglodytes) or a gorilla (Gorilla), which has an amino acid sequence as set forth in SEQ ID NO :13 (MKHVLNLYLLGVVLTLLSIF).

In another embodiment, the truncated sequence of HILPDA may be a truncated product of the corresponding protein derived from a rat (Rattus norvegicus), which has an amino acid sequence as set forth in SEQ ID NO: 14 (MKYMLNLYVLGVMLTLLSIF).

In another embodiment, the truncated sequence of HILPDA may be a truncated product of the corresponding protein derived from a mouse (Mus musculus), which has an amino acid sequence as set forth in SEQ ID NO: 15 (MKFMLNLYVGIMLTLLSIF).

The present inventors have discovered a new application of hypoxia-inducible lipid droplet-associated protein (HILPDA) through research. Since this naturally occurring small protein or polypeptide is derived from the human body itself, it is expected to exhibit low toxicity and lack of immunogenicity when administered to patients, which is suitable for clinical transformation and promotion. Therefore, the activity of HILPDA or a truncated active fragment thereof will be a polypeptide drug with significant medical and commercial value.

In the present invention, the "hyperlipidemia-related disease" refers to a condition characterized by elevated lipid level and their associated complications. These conditions encompass serum total cholesterol levels exceeding 5.72 mmol/L, elevated serum triglyceride levels exceeding 1.70 mmol/L, and reduced serum high-density lipoprotein cholesterol (HDL-C) levels below 0.9 mmol/L, leading to pathological manifestations including but not limited to hyperlipidemia, atherosclerosis stemming from prolonged hyperlipidemia, as well as complications such as cardiovascular diseases, hypertension, hyperglycemia, and hyperuricemia.

In the context of this invention, "hyperlipidemia-related diseases" are defined by elevated lipid level and their associated complications, which include fasting venous serum total cholesterol levels equal to or exceeding 6.2 mmol/L, high serum triglyceride levels equal to or exceeding 2.3 mmol/L, elevated levels of low-density lipoprotein cholesterol (LDL-C) equal to or exceeding 4.1 mmol/L, and decreased serum high-density lipoprotein cholesterol (HDL-C) levels below 1.0 mmol/L. These conditions give rise to pathological symptoms such as hyperlipidemia, atherosclerosis due to prolonged hyperlipidemia, and complications like cardiovascular diseases, hypertension, hyperglycemia, and hyperuricemia.

Within this invention, "neurodegenerative disease" refers to neurological disorders and their associated complications influenced by lipid metabolism within the central nervous system. This category includes, but is not limited to, conditions such as multiple sclerosis, Alzheimer's disease, and Parkinson's disease.

The term "Type III hyperlipidemia" within this invention denotes a hereditary disorder characterized by the abnormal breakdown and clearance of fats (lipids) in the body, resulting in the significant accumulation of certain lipid substances. Pathological symptoms associated with this disease include the presence of yellowish lipid-filled nodules on the skin (xanthomas), pancreatitis (inflammation of the pancreas), and lipid buildup in blood vessels (atherosclerosis). This condition is linked to APOE gene mutations, particularly APOE2.

Usually, the pharmaceutical composition of the present invention contains 0.1% to 90% by weight of the hypoxia-inducible lipid droplet-associated protein or a truncated active fragment thereof as the main drug. Pharmaceutical formulations can be prepared using established methods in the field. For this purpose, if necessary, the main drug can be combined with one or more solid or liquid pharmaceutical excipients and/or adjuvants to prepare an appropriate administration form or dosage form for human use.

The term "adjuvant" in the present application refers to an excipient or vehicle used to administer the main drug, which includes but is not limited to diluent, disintegrating agent, precipitation inhibitor, surfactant, glidant, binder, lubricant, coating material, etc. Excipients are generally described in "Remington's Pharmaceutical Sciences" by E.W. Martin. Examples of excipients include, but are not limited to, aluminum monostearate, aluminum stearate, carboxymethylcellulose, sodium carboxymethylcellulose, crospovidone, glyceryl isostearate, glyceryl monostearate, hydroxyethylcellulose, hydroxymethylcellulose, hydroxyoctacosanyl hydroxystearate, hydroxypropylcellulose, hydroxypropylmethylcellulose, lactose, lactose monohydrate, magnesium stearate, mannitol, microcrystalline cellulose, etc.

The hypoxia-inducible lipid droplet-associated protein or a truncated active fragment thereof can be formulated into pharmaceutical preparations, including dosage forms suitable for oral administration, dosage forms (e.g., solution) suitable for parenteral injection (e.g., intravenous, subcutaneous injection), dosage forms (e.g., ointment, patch, or cream) suitable for topical administration, and dosage forms (e.g., suppository) suitable for rectal administration.

Depending on the disease and patient to be treated and the route of administration, the pharmaceutical composition of the present invention can be administered once or several times per day in different doses. The dosage depends on many factors, such as the severity of the indication to be treated or prevented, the sex, age, body weight and individual response of the patient, the route of administration and the frequency of administration, etc. The above dosage may be administrated in the form of single dose or in the form of divided multiple doses, for example, two, three or four doses.

The actual dosage of the main drug (hypoxia-inducible lipid droplet-associated protein or a truncated active fragment thereof) in the pharmaceutical composition of the present invention can be changed so as to effectively obtain the desired therapeutic response for specific patients, compositions and administration methods. The dosage can be selected based on the route of administration, the severity of the condition to be treated and the condition and previous medical history of the patient to be treated. Typically, it is common practice in this field to initiate dosing below the level required to achieve the desired therapeutic effect and gradually increase the dosage until the desired effect is attained.

The term "effective amount" signifies a dose that can achieve therapeutic, preventive, alleviating, and/or relieving effects for the diseases or conditions described in this invention in a subj ect.

### Brief Description of the Drawings

Figure 1 shows the strategy diagram to evaluate whether HILPDA promotes the uptake of APOE-containing lipoproteins.
Figures 2A to 2D show the APOE uptake by recipient cells in cell culture medium in the presence or absence of HII,PDA; wherein, in Figure 2A, the recipient cells are 786-O cells; in Figure 2B, the recipient cells are 769-P cells; in Figure 2C, the recipient cells are HuH7 cells; and in Figure 2D, the recipient cells are ES-2 cells.
Figure 3 shows the schematic strategy diagram for the synthesized HILPDA or its truncated fragments.
Figures 4A to 4H show the identification results via high-performance liquid chromatography (HPLC) and mass spectrometry for synthesized HILPDA or its truncated fragments.
Figure 5 shows the diagram to evaluate whether the synthesized HILPDA or its truncated fragments promote the APOE-containing lipoproteins.
Figure 6 shows the diagram of APOE uptake by recipient cells in cell culture medium in the presence or absence of HILPDA or its truncated fragments.
Figure 7 shows the strategy diagram to evaluate whether the synthesized HILPDA or its truncated fragments promotes the uptake of lipoproteins by the cells.
Figures 8A to 8D shows the results of lipoprotein uptake by recipient cells in the presence and absence of HII,PDA or its truncated sequences; wherein, the recipient cells in Figure 8A and Figure 8B are HuH-7 cells and Hep-G2 cells; the recipient cells in Figure 8C and Figure 8D are 786-O cells.
Figure 9 shows the diagram to evaluate whether the synthesized HILPDA promotes the reduction of lipoprotein levels in the rat blood.
Figure 10 shows the diagram of the reduction of lipoprotein level in the rat blood under HILPDA treatment.
Figure 11 shows the diagram to evaluate whether the truncated fragments of the synthesized HILPDA promotes the decrease of APOE level in the rat cerebrospinal fluid.
Figure 12 shows the diagram of the reduction of APOE level in the rat cerebrospinal fluid under the treatment with the truncated fragments of HILPDA.
Figure 13 shows the diagram of the APOE uptake by recipient cells in cell culture medium in the presence and absence of HILPDA.
Figure 14 shows the diagram of the APOE uptake in cell culture medium by brain slices in the presence and absence of HILPDA.

### Specific Models for Carrying Out the Invention

Embodiments of the present invention will be described in detail below in conjunction with examples. Those skilled in the art will understand that the following examples are only for illustrating the present invention and should not be considered as limiting the scope of the present invention. For those without indicating specific techniques or conditions in the examples, they were performed according to the techniques or conditions described in documents in this field (e.g., referring to J. Sambrook et al., "Molecular Cloning Experiment Guide", translated by Huang Peitang, third edition, Science Press) or following the product instructions. For the reagents or instruments used without indicating manufacturers, they were all commercially available conventional products.

### Example 1: Construction of eukaryotic expression plasmid expressing HILPDA-Flag fusion protein

1) The DNA sequence encoding the human HILPDA-Flag fusion protein (HILPDA protein fused with FLAG tag) was directly synthesized by GENEWIZ (Genewiz Inc.) as follows:
   DNA sequence: 
ATGAAGCATGTGTTGAACCTCTACCTGTTAGGTGTGGTACTGACCCTACTCTCC
ATCTTCGTTAGAGTGATGGAGTCCCTAGAGGGCTTACTAGAGAGCCCATCGCCTGGG
ACCTCCTGGACCACCAGAAGCCAACTAGCCAACACAGAGCCCACCAAGGGCCTTCC
AGACCATCCATCCAGAAGCATGGATTACAAGGATGACGACGATAAGTGA (SEQ ID
NO: 15).
2) Design of corresponding primers
   Forward primer: ATAGAAGATTCTAGAATGAAGCATGTGTTGAACCTCTAC (SEQ ID NO: 16);
   Reverse primer: CGCGGCCGCGGATCCtcaCTTATCGTCGTCATCCTTGTAATC (SEQ ID NO: 17).

The above-mentioned synthesized DNA sequence was used as a template for PCR amplification to obtain the target fragment (the underlined parts indicated the homologous arms of the vector).
3) The eukaryotic expression vector pCDH-CMV-MCS-EF1-puro (Cat. No.: VT1480, Youbao Biological) was linearized with the restriction endonucleases XbaI (Cat. No.: R0145L, NEB) and BamHI (Cat. No.: R3136L, NEB).
4) The homologous recombination method mediated by Exonuclease III (Cat. No.: 2170A, TAKARA) was used to ligate the target fragment and the digested linear vector, and the ligated product was transformed into *E. coli* DH5-α, and positive colony clones were obtained on Ampicillin-resistant LB solid medium plates.
5) The positive colony clones were amplified, and plasmids were extracted using a plasmid purification kit.
6) After sequencing verification, the correct HILPDA fusion protein expression plasmid was obtained.

### Example 2: Construction of GFP-Flag fusion protein expression plasmid

1) GFP gene sequence was derived from the vector pCDH-CMV-MCS-EF1-GFP+Puro (Cat. No.: VT8070, Youbao Biological), and its specific sequence was as follows:

ATGGTGAGCAAGGGCGAGGAGCTGTTCACCGGGGTGGTGCCCATCCTGGTCGA
GCTGGACGGCGACGTAAACGGCCACAAGTTCAGCGTGTCCGGCGAGGGCGAGGGCG
ATGCCACCTACGGCAAGCTGACCCTGAAGTTCATCTGCACCACCGGCAAGCTGCCCG
TGCCCTGGCCCACCCTCGTGACCACCCTGACCTACGGCGT
GCAGTGCTTCAGCCGCTACCCCGACCACATGAAGCAGCACGACTTCTTCAAGTCCGC
CATGCCCGAAGGCTACGTCCAGGAGCGCACCATCTTCTTCAAGGACGACGGCAAC
TACAAGACCCGCGCCGAGGTGAAGTTCGAGGGCGACACCCTGGTGAACCGCATCGA
GCTGAAGGGCATCGACTTCAAGGAGGACGGCAACATCCTGGGGCACAAG
CTGGAGTACAACTACAACAGCCACAACGTCTATATCATGGCCGACAAGCAGAAGAA
CGGCATCAAGGTGAACTTCAAGATCCGCCACAACATCGAGGACGGCAGCGTGCAGC
TCGCCGACCACTACCAGCAGAACACCCCCATCGGCGACGGCCCCGTGCTGCTGCCC
GACAACCACTACCTGAGCACCCAGTCCGCCCTGAGCAAAGACCCCAACGAGAAGC
GCGATCACATGGTCCTGCTGGAGTTCGTGACCGCCGCCGGGATCACTCTCGGCATGG
ACGAGCTGTACAAGtaa (SEQ ID NO: 18).

In the above sequence of SEQ ID NO: 18, the lowercase letters represented stop codon.

### 2) Design of corresponding primers

Forward primer: ATAGAAGATTCTAGAATGGTGAGCAAGGGCGAGGA (SEQ ID NO: 19);
Reverse primer: CGCGGCCGCGGATCCtta**CTTATCGTCGTCATCCTTGTAATC**CTT GTACAGCTCGTCCATGC (SEQ ID NO: 20).

The above GFP DNA sequence was used as a template to perform PCR amplification to obtain the target fragment GFP-Flag sequence (the underlined parts indicated the homology arms of the vector, and the bolded part indicated the Flag sequence).
3) Eukaryotic expression vector pCDH-CMV-MCS-EF1-puro (Cat. No.: VT1480, Youbao Biological) was digested with restriction endonucleases XbaI (Cat. No.: R0145L, NEB) and BamHI (Cat. No.: R3136L, NEB) to obtain a linear vector.
4) The homologous recombination method mediated by Exonuclease III (Cat. No.: 2170A, TAKARA) was used to ligate the target fragment and the digested linear vector, and the ligated product was transformed into *E. coli* DH5α, and positive colony clones were obtained on Ampicillin-resistant LB solid medium plates.
5) The positive colony clones were amplified, and plasmids were extracted using a plasmid purification kit.
6) After sequencing verification, the correct GFP-Flag fusion protein expression plasmid was obtained.

### Example 3: Construction of APOE2-3xHA, APOE3-3xHA and APOE4-3xHA fusion protein expression plasmids

1) APOE3 gene sequence was derived from the vector pDONR223-APOE (Cat. No.: G118334, Youbao Biological), and its specific sequence was as follows:

ATGAAGGTTCTGTGGGCTGCGTTGCTGGTCACATTCCTGGCAGGATGCCAGGCC
AAGGTGGAGCAAGCGGTGGAGACAGAGCCGGAGCCCGAGCTGCGCCAGCAGACCG
AGTGGCAGAGCGGCCAGCGCTGGGAACTGGCACTGGGTCGCTTTTGGGATTACCTG
CGCTGGGTGCAGACACTGTCTGAGCAGGTGCAGGAGGAGCTGCTCAGCTCCCAGGT
CACCCAGGAACTGAGGGCGCTGATGGACGAGACCATGAAGGAGTTGAAGGCCTACA
AATCGGAACTGGAGGAACAACTGACCCCGGTGGCGGAGGAGACGCGGGCACGGCT
GTCCAAGGAGCTGCAGGCGGCGCAGGCCCGGCTGGGCGCGGACATGGAGGACGTGT
GCGGCCGCCTGGTGCAGTACCGCGGCGAGGTGCAGGCCATGCTCGGCCAGAGCACC
GAGGAGCTGCGGGTGCGCCTCGCCTCCCACCTGCGCAAGCTGCGTAAGCGGCTCCTC
CGCGATGCCGATGACCTGCAGAAGCGCCTGGCAGTGTACCAGGCCGGGGCCCGCGA
GGGCGCCGAGCGCGGCCTCAGCGCCATCCGCGAGCGCCTGGGGCCCCTGGTGGAAC
AGGGCCGCGTGCGGGCCGCCACTGTGGGCTCCCTGGCCGGCCAGCCGCTACAGGAG
CGGGCCCAGGCCTGGGGCGAGCGGCTGCGCGCGCGGATGGAGGAGATGGGCAGCC
GGACCCGCGACCGCCTGGACGAGGTGAAGGAGCAGGTGGCGGAGGTGCGCGCCAA
GCTGGAGGAGCAGGCCCAGCAGATACGCCTGCAGGCCGAGGCCTTCCAGGCCCGCC
TCAAGAGCTGGTTCGAGCCCCTGGTGGAAGACATGCAGCGCCAGTGGGCCGGGCTG
GTGGAGAAGGTGCAGGCTGCCGTGGGCACCAGCGCCGCCCCTGTGCCCAGCGACAA
TCACtga (SEQ ID NO: 21 ).

In the above sequence of SEQ ID NO: 21, the lowercase letters indicated stop codon, and the underlined parts represented the distinguishing sites of different alleles of APOE, and in this sequence, it specifically referred to APOE3.

### 2) Design of corresponding APOE3 primers

Forward primer: ATAGAAGATTCTAGAATGAAGGTTCTGTGGGCTGCG (SEQ ID NO: 22);
Reverse primer: CTTCCATGGCTCGAGGTGATTGTCGCTGGGCACAG (SEQ ID NO: 23).

The above DNA sequence of APOE3 was used as a template to carry out PCR amplification to obtain the target fragment APOE3 sequence (the underlined parts indicated the homologous arms of the vector).

3) The gene sequences of APOE2 and APOE4 were obtained by point mutations based on the APOE3 gene sequence.
(1) Design of point mutation primers
   APOE2 forward primer: ATGCCGATGACCTGCAGAAGTGCCTGGCAGTGTACCAG GC (SEQ ID NO: 24),
   APOE2 reverse primer: GCCTGGTACACTGCCAGGCACTTCTGCAGGTCATCGGCAT (SEQ ID NO: 25);
   APOE4 forward primer: GCGCGGACATGGAGGACGTGCGCGGCCGCCTGGTGC AGTA (SEQ ID NO: 26),
   APOE4 reverse primer: TACTGCACCAGGCGGCCGCGCACGTCCTCCATGTCCGCGC (SEQ ID NO: 27)

4) By using the APOE3 forward primer + APOE2 reverse primer, the APOE3 gene sequence was used as a template to perform PCR amplification to obtain Fragment 1; and in the meantime, by using the APOE2 forward primer + APOE3 reverse primer, the APOE3 gene sequence was used as a template to perform PCR amplification to obtain Fragment 2. Finally, by using the APOE3 forward primer+APOE3 reverse primer, the mixture of Fragment 1 + Fragment 2 was used as a template to perform PCR amplification to obtain the sequence of the target fragment APOE2. The specific sequence of APOE2 was as follows:

ATGAAGGTTCTGTGGGCTGCGTTGCTGGTCACATTCCTGGCAGGATGCCAGGCC
AAGGTGGAGCAAGCGGTGGAGACAGAGCCGGAGCCCGAGCTGCGCCAGCAGACCG
AGTGGCAGAGCGGCCAGCGCTGGGAACTGGCACTGGGTCGCTTTTGGGATTACCTG
CGCTGGGTGCAGACACTGTCTGAGCAGGTGCAGGAGGAGCTGCTCAGCTCCCAGGT
CACCCAGGAACTGAGGGCGCTGATGGACGAGACCATGAAGGAGTTGAAGGCCTACA
AATCGGAACTGGAGGAACAACTGACCCCGGTGGCGGAGGAGACGCGGGCACGGCT
GTCCAAGGAGCTGCAGGCGGCGCAGGCCCGGCTGGGCGCGGACATGGAGGACGTGT
GCGGCCGCCTGGTGCAGTACCGCGGCGAGGTGCAGGCCATGCTCGGCCAGAGCACC
GAGGAGCTGCGGGTGCGCCTCGCCTCCCACCTGCGCAAGCTGCGTAAGCGGCTCCTC
CGCGATGCCGATGACCTGCAGAAGTGCCTGGCAGTGTACCAGGCCGGGGCCCGCGA
GGGCGCCGAGCGCGGCCTCAGCGCCATCCGCGAGCGCCTGGGGCCCCTGGTGGAAC
AGGGCCGCGTGCGGGCCGCCACTGTGGGCTCCCTGGCCGGCCAGCCGCTACAGGAG

CGGGCCCAGGCCTGGGGCGAGCGGCTGCGCGCGCGGATGGAGGAGATGGGCAGCC
GGACCCGCGACCGCCTGGACGAGGTGAAGGAGCAGGTGGCGGAGGTGCGCGCCAA
GCTGGAGGAGCAGGCCCAGCAGATACGCCTGCAGGCCGAGGCCTTCCAGGCCCGCC
TCAAGAGCTGGTTCGAGCCCCTGGTGGAAGACATGCAGCGCCAGTGGGCCGGGCTG
GTGGAGAAGGTGCAGGCTGCCGTGGGCACCAGCGCCGCCCCTGTGCCCAGCGACAA
TCACtga (SEQ ID NO: 28).

In the above sequence of SEQ ID NO: 28, the lowercase letters indicated stop codon, and the underlined parts represented the distinguishing sites of different alleles of APOE, and in this sequence, it specifically referred to APOE2.

5) By using the APOE3 forward primer + APOE4 reverse primer, the APOE3 gene sequence was used as a template to perform PCR amplification to obtain Fragment 1; in the meantime, by using the APOE4 forward primer + APOE3 reverse primer, the APOE3 gene sequence was used as a template to perform PCR amplification to obtain Fragment 2. Finally, by using the APOE3 forward primer + APOE3 reverse primer, the mixture of Fragment 1 + Fragment 2 was used as a template to perform PCR amplification to obtain the sequence of the target fragment APOE4. The specific sequence of APOE4 was as follows:

ATGAAGGTTCTGTGGGCTGCGTTGCTGGTCACATTCCTGGCAGGATGCCAGGCC
AAGGTGGAGCAAGCGGTGGAGACAGAGCCGGAGCCCGAGCTGCGCCAGCAGACCG
AGTGGCAGAGCGGCCAGCGCTGGGAACTGGCACTGGGTCGCTTTTGGGATTACCTG
CGCTGGGTGCAGACACTGTCTGAGCAGGTGCAGGAGGAGCTGCTCAGCTCCCAGGT
CACCCAGGAACTGAGGGCGCTGATGGACGAGACCATGAAGGAGTTGAAGGCCTACA
AATCGGAACTGGAGGAACAACTGACCCCGGTGGCGGAGGAGACGCGGGCACGGCT
GTCCAAGGAGCTGCAGGCGGCGCAGGCCCGGCTGGGCGCGGACATGGAGGACGTG
CGCGGCCGCCTGGTGCAGTACCGCGGCGAGGTGCAGGCCATGCTCGGCCAGAGCAC
CGAGGAGCTGCGGGTGCGCCTCGCCTCCCACCTGCGCAAGCTGCGTAAGCGGCTCCT
CCGCGATGCCGATGACCTGCAGAAGCGCCTGGCAGTGTACCAGGCCGGGGCCCGCG
AGGGCGCCGAGCGCGGCCTCAGCGCCATCCGCGAGCGCCTGGGGCCCCTGGTGGAA
CAGGGCCGCGTGCGGGCCGCCACTGTGGGCTCCCTGGCCGGCCAGCCGCTACAGGA
GCGGGCCCAGGCCTGGGGCGAGCGGCTGCGCGCGCGGATGGAGGAGATGGGCAGC
CGGACCCGCGACCGCCTGGACGAGGTGAAGGAGCAGGTGGCGGAGGTGCGCGCCA
AGCTGGAGGAGCAGGCCCAGCAGATACGCCTGCAGGCCGAGGCCTTCCAGGCCCGC
CTCAAGAGCTGGTTCGAGCCCCTGGTGGAAGACATGCAGCGCCAGTGGGCCGGGCT
GGTGGAGAAGGTGCAGGCTGCCGTGGGCACCAGCGCCGCCCCTGTGCCCAGCGACA
ATCACtga (SEQ ID NO: 29).

In the above sequence SEQ ID NO: 29, the lowercase letters indicated stop codon, and the underlined parts represented the distinguishing sites of different alleles of APOE, and in this sequence, it specifically referred to APOE4.
6) Eukaryotic expression vector pCDH-CMV-MCS-EF1-3xHA-puro (self-assembled on the above pCDH-CMV-MCS-EF1-puro vector by the method as follows) was double digested by using restriction endonucleases XbaI (Cat. No.: R0145L, NEB) and XhoI (Cat. No.: R0146L, NEB) to obtain a linear vector. The construction of the vector pCDH-CMV-MCS-EF 1-3xHA-puro was as follows: first, the vector pCDH-CMV-MCS-EF1-puro was double digested with XhoI (Cat. No.: R0145L, NEB) and NotI (Cat. No.: R3189L, NEB) to obtain a linear vector, and then the 3xHA sequence (TACCCTTATGACGTACCTGACTATGCTGGCGCCTACCCTTATGACGTACCTGACTAT GCTGGATACCCTTATGACGTACCTGACTATGCTtaa, SEQ ID NO: 30) was ligated with the linear vector pCDH-CMV-MCS-EF1-puro to form a vector with a 3xHA tag at the carboxyl terminal, namely pCDH-CMV-MCS-EF1-3xHA-puro.
7) The homologous recombination method mediated by Exonuclease III (Cat. No.: 2170A, TAKARA) was used to ligate the target fragment APOE2, APOE3 or APOE4 with the digested linear vector pCDH-CMV-MCS-EF1-3xHA-puro, and the ligated product was transformed into *E*. *coli* DH5α, and positive colony clones were obtained on Ampicillin-resistant LB solid medium plates.
8) The positive colony clones were amplified, and plasmids were extracted by using a plasmid purification kit.
9) After sequencing verification, the correct APOE2-3xHA, APOE3-3xHA and APOE4-3xHA fusion protein expression plasmids were obtained.

### Example 4: Clearance of apolipoprotein and lipoprotein promoted by secretory HILPDA in cell culture medium

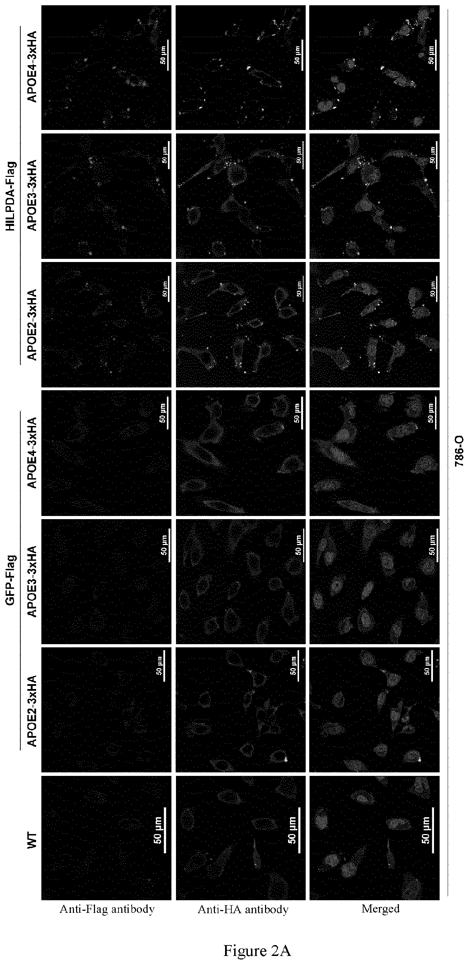

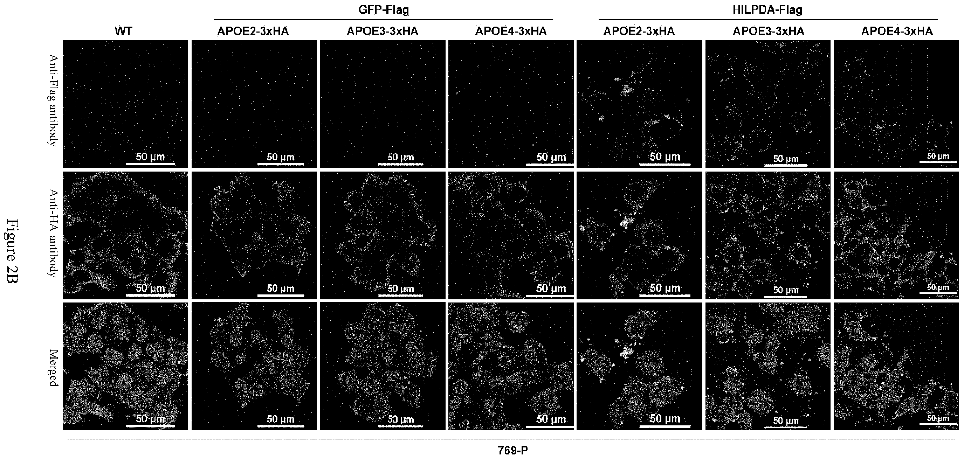

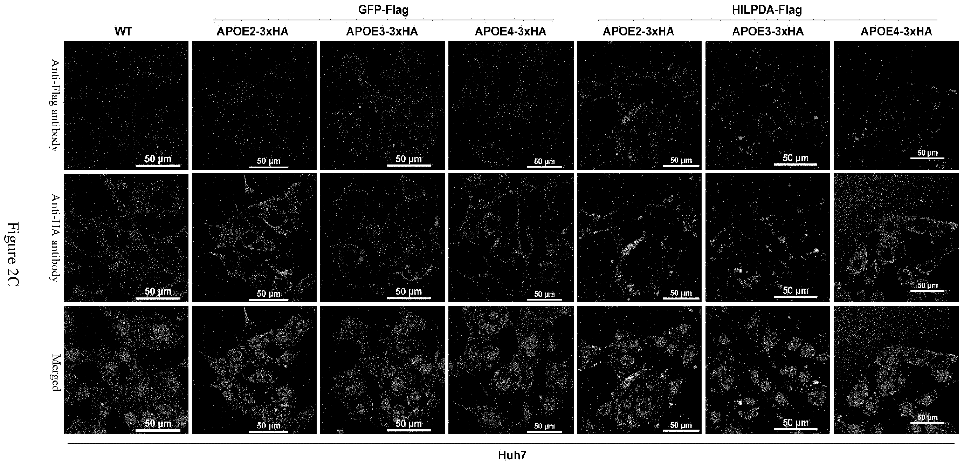

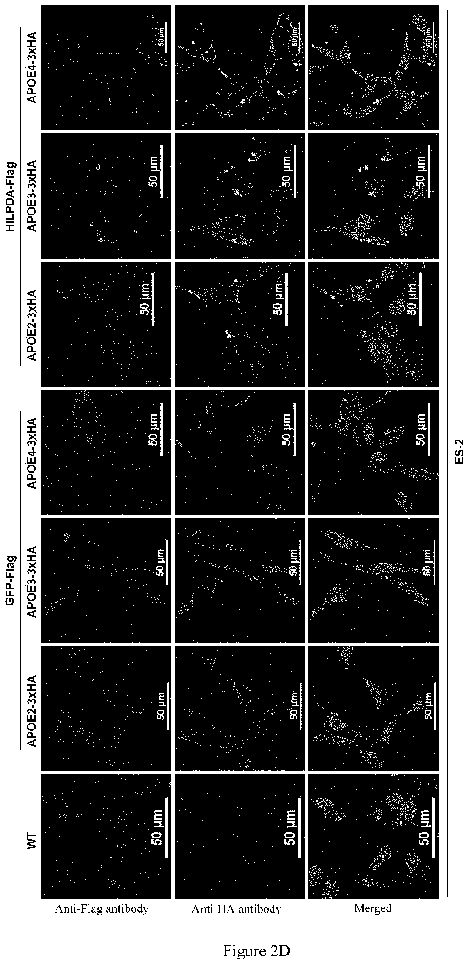

1) HEK293T cells were cultured with DMEM medium in a 10cm cell culture dish.
2) The above cells were separately co-transfected with:
   GFP-Flag expressing plasmid and APOE2-3xHA plasmid,
   GFP-Flag expressing plasmid and APOE3-3xHA plasmid,
   GFP-Flag expressing plasmid and APOE4-3xHA plasmid,
   HILPDA-Flag expressing plasmid and APOE2-3xHA plasmid,
   HILPDA-Flag expressing plasmid and APOE3-3xHA plasmid, and
   HILPDA-Flag expressing plasmid and APOE4-3xHA plasmid.
3) After 12 hours, the old medium in 1) was replaced with fresh DMEM cell culture medium.
4) After further culturing for 48 hours, the media of the cultured cells in 3) were collected, respectively, and these media were used to directly culture the recipient cells, such as 786-O (Cat. No.: Cell Bank TCHu186, Cell Bank of Type Culture Collection Committee of Chinese Academy of Sciences/Cell Resource Center of Shanghai Institutes for Life Sciences, Chinese Academy of Sciences), 769-P (Cat. No.: Cell Bank TCHu215, Cell Bank of Type Culture Collection Committee of Chinese Academy of Sciences/Cell Resource Center of Shanghai Institutes for Life Sciences, Chinese Academy of Sciences), HuH7 (Cat. No.: TCHu182, Cell Bank of Type Culture Collection Committee of Chinese Academy of Sciences/Cell Resource Center of Shanghai Institutes for Life Sciences, Chinese Academy of Sciences), ES-2 (Cat. No.: TCHu111, Cell Bank of Type Culture Collection Committee of Chinese Academy of Sciences/Cell Resource Center of Shanghai Institutes for Life Sciences, Chinese Academy of Sciences) wild-type cells, that had been pre-digested with trypsin containing 0.25% EDTA.
5) After culturing for 24 hours, anti-Flag (Cat. No.: F1804, sigma; for detecting GFP-Flag or HILPDA-Flag fusion protein) and anti-HA antibody (Cat. No.: ab9110, abcam; for detecting fusion proteins expressed by three different alleles of APOE2-3xHA, APOE3-3xHA or APOE4-3xHA) were used to perform immunofluorescent staining experiments.
6) The localization of APOE-3xHA and HILPDA-Flag was observed by confocal laser microscopy (as shown in Figures 2A to 2D).

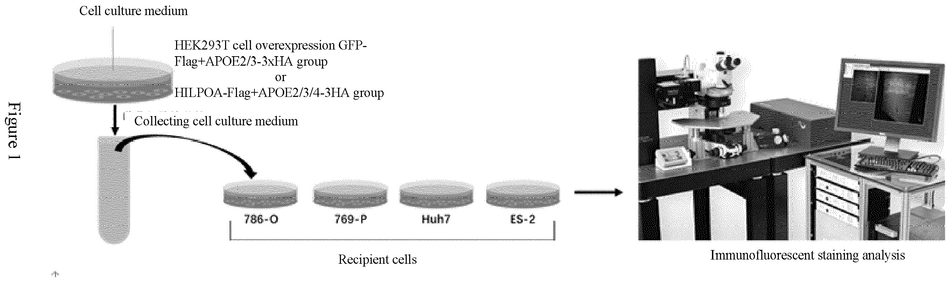

Figure 1 shows the strategy to evaluate whether HILPDA promoted the uptake of lipoproteins formed with APOE. Figures 2A to 2D show the APOE uptake by the recipient cells in cell culture medium in the presence and absence of HILPDA.

The experimental results showed that: compared with the recipient cells (e.g., 786-O, 769-P, Huh7 or ES-2) cultured in the culture medium of HEK293T cells co-expressing GFP-Flag and APOE2-3xHA protein, GFP-Flag and APOE3-3xHA protein, or GFP-Flag and APOE4-3xHA protein, the recipient cells cultured in the culture medium of HEK293T cells co-expressing HILPDA-Flag and APOE2-3xHA protein, HILPDA-Flag and APOE3-3xHA protein, or HILPDA-Flag and APOE4-3xHA protein showed more and stronger fluorescent signals, indicating that in the presence of HILPDA, the recipient cells absorbed more apolipoprotein APOE2, APOE3 or APOE4 (anti-HA antibody group indicated), also suggesting that HILPDA promoted the clearance of apolipoproteins and lipoproteins formed thereby in the culture medium or serum (theoretically, APOE2-3xHA, APOE3-3xHA and APOE4-3xHA existed in the form of lipoproteins).

### Example 5: In vitro synthesis of HILPDA-related polypeptides

1) The following peptide sequences were synthesized by peptide solid-phase synthesis on Rink Amide AM resin (substitution degree: 0.15 mmol/g):
   HILPDA P01:
      MKFMLNLYVGIMLTLLSIFVRVMESLGGLLESPLPGSSWITRGQLANTQPPKGLPD HPSRGVQ (SEQ ID NO: 2)
   HILPDA P02:
      MKYMLNLYVLGVMLTLLSIFVRLMESLGGLLENPLPGSSWITRGHLANTQPPRGLP DHPSRGVQ (SEQ ID NO: 12)
   HILPDA P03: MKHVLNLYLLGVVLTLLSIF (SEQ ID NO: 13)
   HILPDA P04: MKYMLNLYVLGVMLTLLSIF (SEQ ID NO: 14)
2) TFA/iPr₃SiH/H₂O (90:5:5 vol%) was used to cleave the synthesized peptides from the resin, and the cleavage process was carried out at room temperature for 2 hours.
3) The lysate was collected and concentrated under nitrogen protection.
4) Then it was cooled with Et₂O to precipitate a crude peptide.
5) After centrifugation (10 minutes, 4°C, 6800 rcf), the precipitation process in 4) was repeated.
6) The crude product was purified by HPLC (Pump A: 0.1 vol% TFA, 100% water; Pump B: 0.1 vol% TFA, 100% acetonitrile; 1 mL/min; 214 nm monitoring).
7) Appropriate fractions were combined and lyophilized to obtain the polypeptide as a white solid.

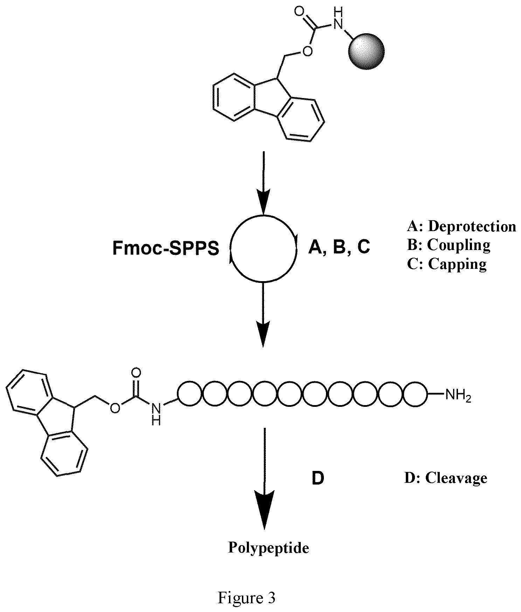

Figure 3 shows a schematic diagram of the synthesis method of HILPDA or its truncated sequence.

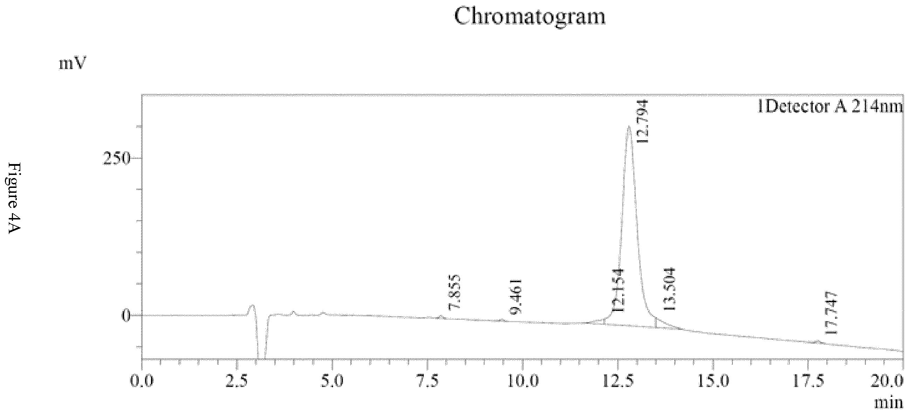

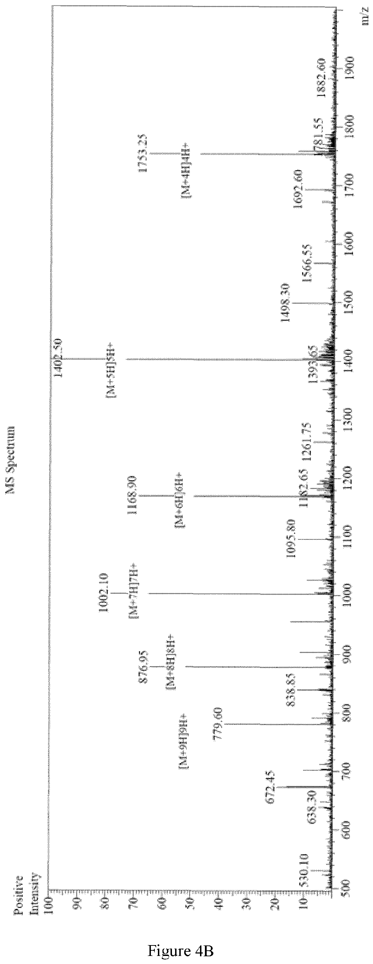

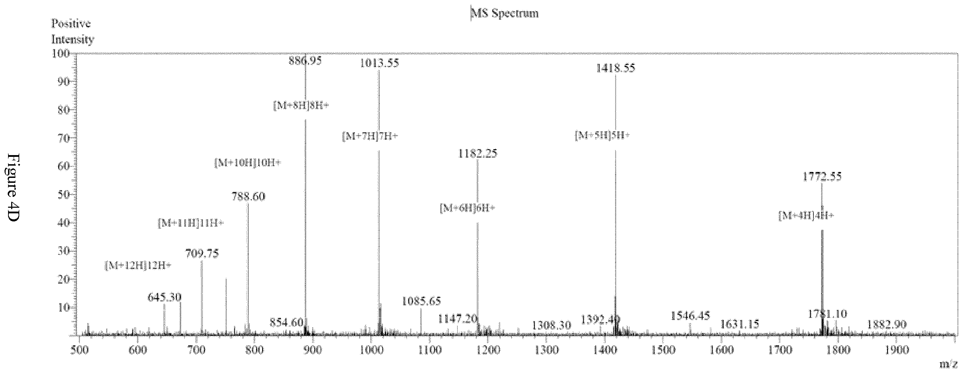

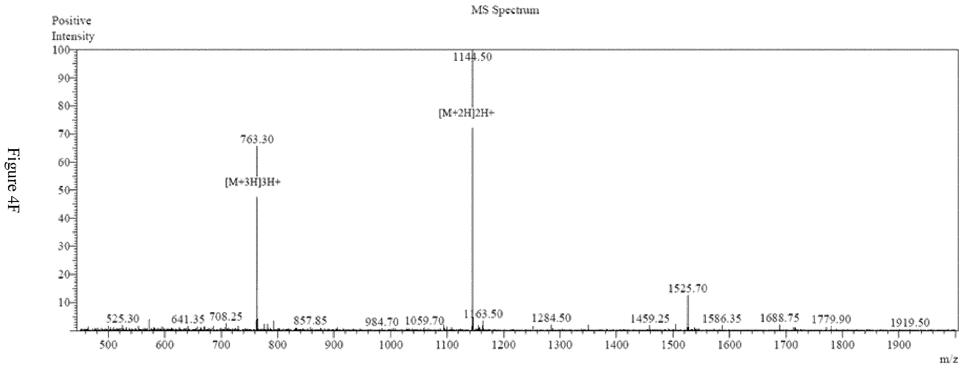

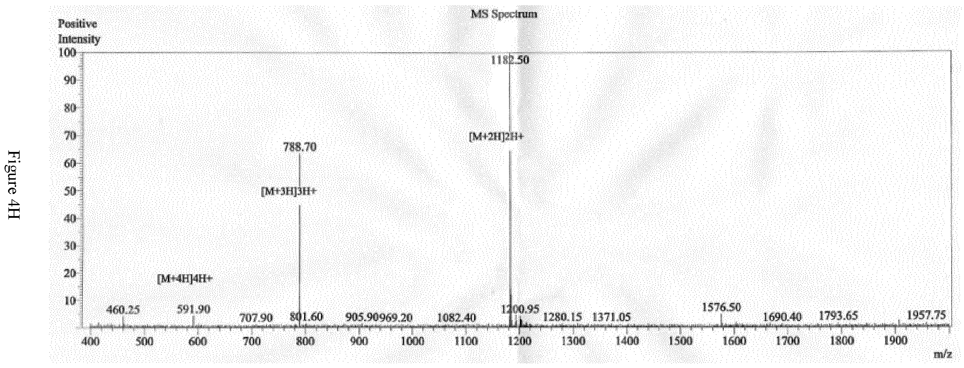

Figures 4A to 4B show the detection results of high-performance liquid chromatography (HPLC) and mass spectrometry of the synthesized HILPDA P01.

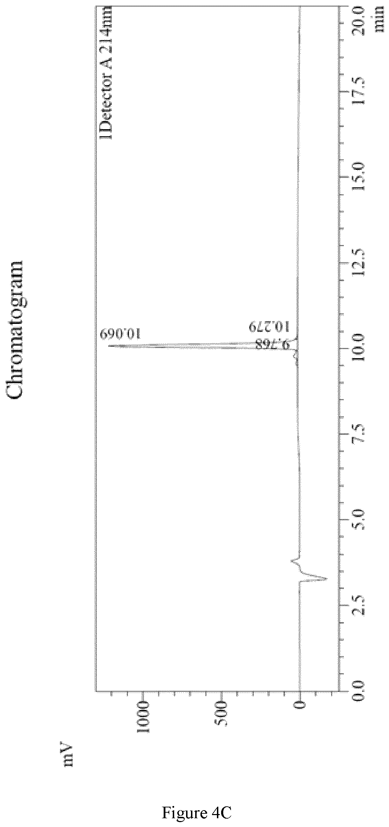

Figures 4C to 4D show the detection results of high-performance liquid chromatography (HPLC) and mass spectrometry of the synthesized HILPDA P02.

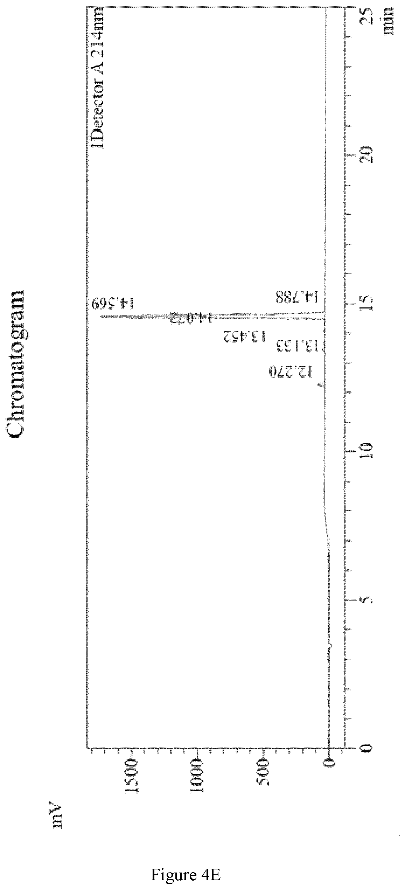

Figures 4E to 4F show the detection results of high-performance liquid chromatography (HPLC) and mass spectrometry of the synthesized truncated sequence HILPDA P03.

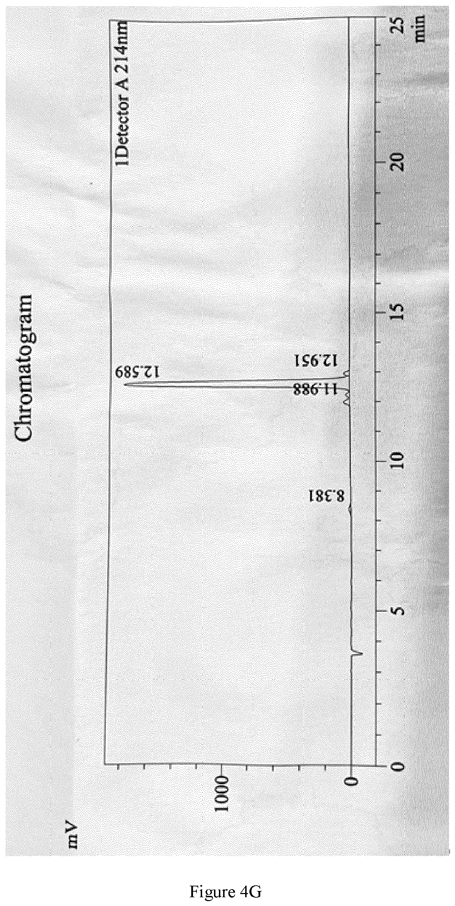

Figures 4G to 4H show the detection results of high-performance liquid chromatography (HPLC) and mass spectrometry of the synthesized truncated sequence HILPDA P04.

### Example 6: HILPDA P01 and HILPDA P03 promote the clearance of apolipoprotein and lipoprotein from cell medium

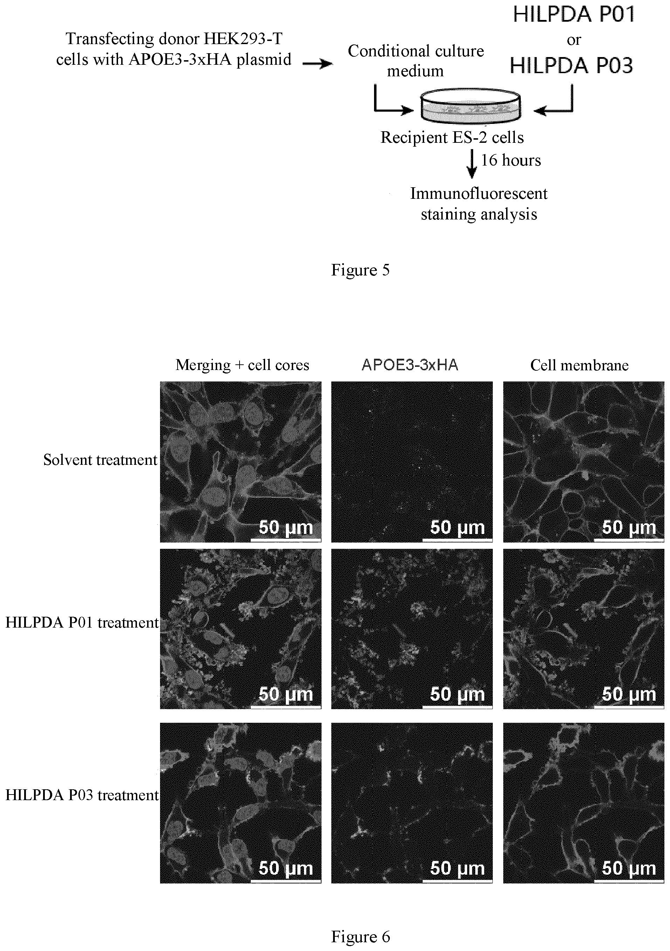

1) HEK293T cells were cultured with DMEM medium in a 10cm cell culture dish.
2) A plasmid of APOE3-3xHA gene was expressed in the above cells.
3) After 12 hours, the old medium in 1) was replaced with fresh DMEM cell culture medium.
4) After further culturing for 48 hours, the media of the cells cultured in 3) were collected, respectively, and these media were used to directly culture the recipient ES-2 cells (Cat. No.: TCHu111, Cell Bank of Typical Culture Collection Committee of Chinese Academy of Sciences/Cell Resource Center of Shanghai Institutes for Life Sciences, Chinese Academy of Sciences) that had been pre-digested with 0.25% EDTA trypsin, and at the same time, 10 µM truncated sequence HILPDA P01, HILPDA P03 or the corresponding volume of solvent (DMSO) was added to the cell culture media.
5) After culturing for 24 hours, anti-HA antibody (Cat. No.: ab9110, abcam; a fusion protein for detecting APOE3-3xHA gene expression) was used to carry out immunofluorescent staining experiments.
6) The uptake of APOE3-3xHA was observed by laser confocal microscope (as shown in Figure 6).

Figure 5 shows the strategy to evaluate whether the synthesized HILPDA or its truncated sequences promoted the uptake of lipoproteins formed with APOE. Figure 6 shows the APOE uptake by the recipient cells in cell culture medium in the presence and absence of HILPDA.

The experimental results showed that: compared with the solvent treatment group, stronger fluorescent signals appeared in the recipient cells treated with HILPDA or its truncated sequences, indicating that the recipient cells absorbed more APOE apolipoprotein in the presence of HILPDA, and suggesting that the synthesized HILPDA or its truncated sequences could promote the clearance of lipoproteins in cell culture medium or serum.

### Example 7: HILPDA P01 and HILPDA P03 promote the uptake of purified human low-density lipoprotein in cells

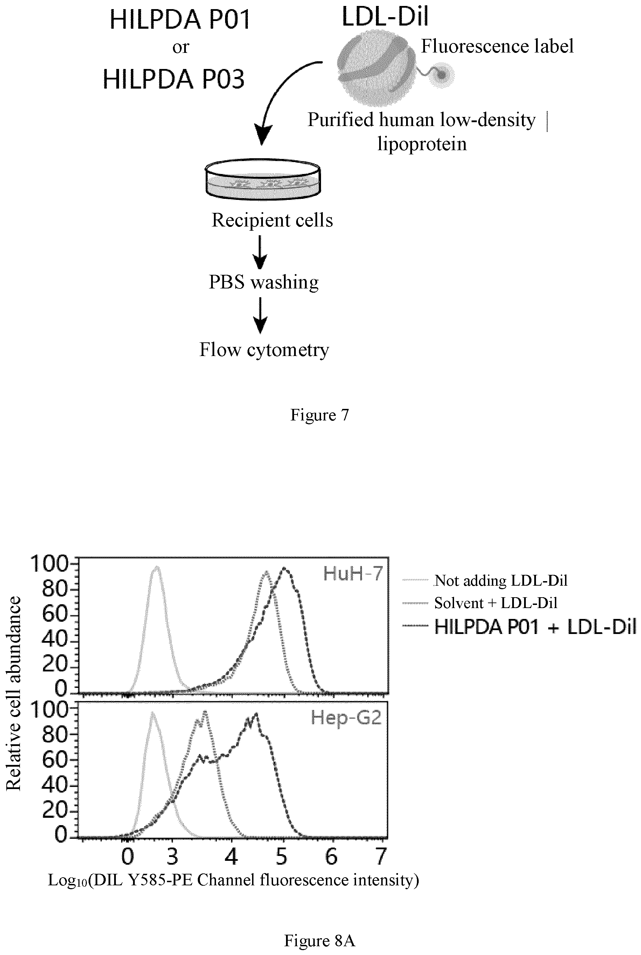

1) HuH-7 cells (Cat. No.: TCHu182, Cell Bank of Type Culture Collection Committee of Chinese Academy of Sciences/Cell Resource Center of Shanghai Institutes for Life Sciences, Chinese Academy of Sciences) and Hep-G2 cells (Cat. No.: HB-8065, ATCC, USA) or 786-O cells (Cat. No.: Cell Bank TCHu186, Cell Bank of Type Culture Collection Committee of Chinese Academy of Sciences/Cell Resource Center of Shanghai Institutes for Life Sciences, Chinese Academy of Sciences) were cultured with DMEM medium in a 10cm cell culture dish.
2) Low-density lipoprotein from human plasma and Dil complex (Cat. No. L3482, Invitrogen) were added at a final concentration of 5 µg/mL to the above cells, and at the same time, 10 µM HILPDA P01, truncated sequence P03 of HILPDA or corresponding volume of solvent (DMSO) was added to the cell culture medium.
3) After culturing for 3 hours, the cells were washed twice with PBS, and the flow cytometry experiment was performed, and the Dil signal intensity of the cells was detected on the PE channel (as shown in Figures 8A to 8D).
Figure 7 shows the strategy to evaluate whether HILPDA or its truncated sequences promoted the uptake of human low-density lipoprotein particles by the cells.
Figures 8A to 8D show the uptake of human low-density lipoprotein particles by the recipient cells in the cell culture medium in the presence and absence of HILPDA or its truncated sequences; wherein:

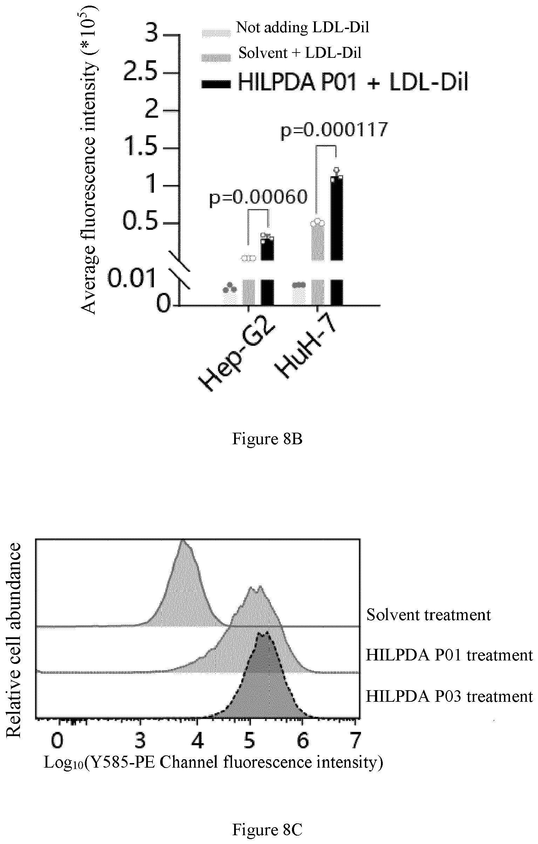

Figures 8A to 8B show the uptake of human low-density lipoprotein particles by HuH-7 and HepG2 cells in the cell culture medium in the presence or absence of HILPDA P01, and Figure 8B shows the quantitative analysis of Figure 8A;

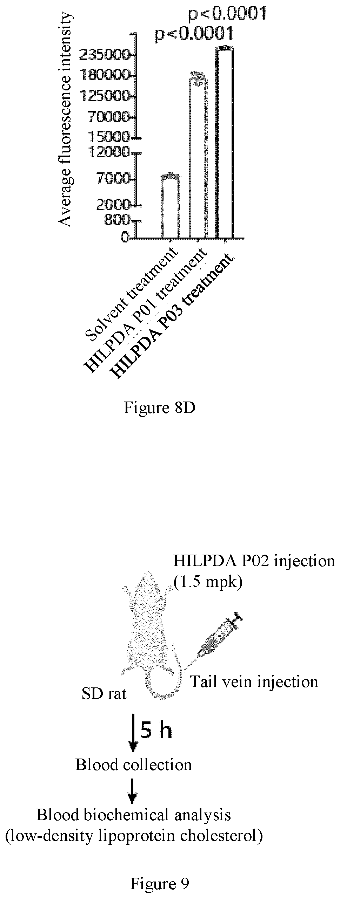

Figures 8C to 8D show the uptake of human low-density lipoprotein particles by 786-O cells in cell culture medium in the presence or absence of HILPDA P01 and truncated sequence P03 of HILPDA, and Figure 8D shows the quantitative analysis of Figure 8C.

The experimental results showed that: compared with the solvent treatment group, stronger fluorescent signals appeared in the recipient cells treated with synthetic HILPDA or its truncated sequences, indicating that the recipient cells absorbed more human low-density lipoprotein particles in the presence of HILPDA, and suggesting that synthetic HILPDA or its truncated sequences could promote the clearance of lipoproteins in serum.

### Example 8: HILPDA P02 decreases the low-density lipoprotein cholesterol level in rat blood

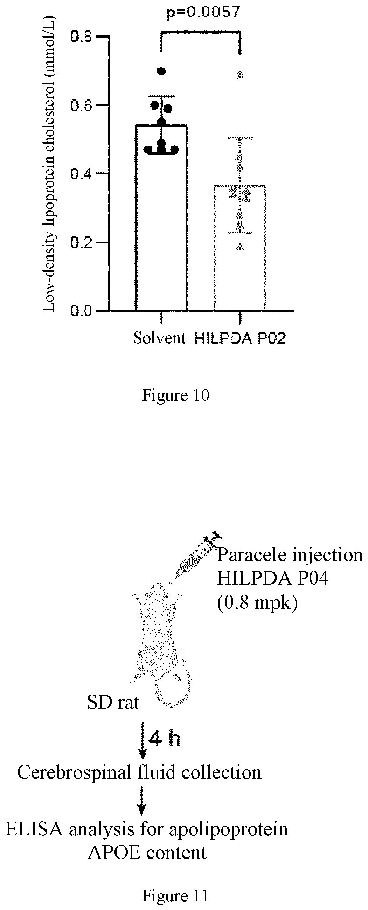

1) SD rats (8-10 weeks old, female, Shanghai Family Planning Institute) were injected through tail vein with HILPDA P02 at a dose of 1.5 mg/kg rat body weight or the same amount of solvent (50% DMSO + 50% 0.9% NaCl) in total of 100 µL;
2) After 5 hours of injection, about 1mL of blood was collected from the tail vein, and the collected blood was placed in an EDTA-K2 anticoagulant tube and underwent ice-bath for 1 hour, then centrifuged at 2000*g at 4°C for 10 minutes, and the supernatant plasma was collected;
3) A biochemical analyzer (Mindray 240) was used to analyze the low-density lipoprotein cholesterol content in the plasma (as shown in Figure 10).
Figure 9 shows the strategy to evaluate whether HILPDA promoted the decrease of low-density lipoprotein cholesterol level in plasma.
Figure 10 shows the comparison of low-density lipoprotein cholesterol level in plasma after injection of solvent or HILPDA P02.

The experimental results showed that compared with the solvent treatment group, the low-density lipoprotein cholesterol level in plasma of the rats treated with HILPDA P02 was significantly reduced, indicating that HILPDA promoted the clearance of lipoproteins in the blood.

### Example 9: HILPDA P04 decreases the APOE level in rat cerebrospinal fluid

1) SD rats (8-10 weeks, male, Shanghai Family Planning Institute) were injected through lateral ventricle with the truncated sequence P04 of HILPDA at a dose of 0.8 mg/kg rat body weight or with the same amount of solvent (50% DMSO+50% 0.9%NaCl) in total of 20 µL;
2) After 4 hours of injection, the cerebrospinal fluid of the rat was collected;
3) ELISA kit (Cat. No.: E-EL-R1230c, ELabScience) was used to detect the apolipoprotein APOE content in the cerebrospinal fluid.
Figure 11 shows the strategy for assessing whether HILPDA promoted the reduction of apolipoprotein APOE level in the cerebrospinal fluid.

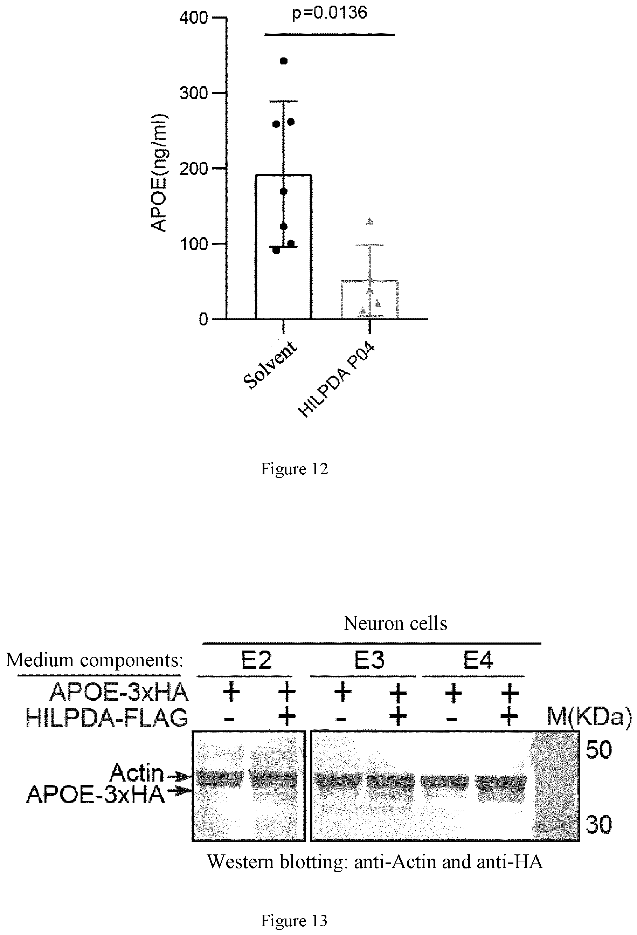

Figure 12 shows the comparison of the apolipoprotein APOE content in the cerebrospinal fluid after injection with the solvent or the truncated sequence P04 of HILPDA.

The experimental results showed that: compared with the solvent treatment group, the content of apolipoprotein APOE in the cerebrospinal fluid of rats treated with the truncated sequence HILPDA P04 was significantly decreased, indicating that HILPDA promoted the clearance of lipoproteins in the cerebrospinal fluid.

### Example 10: Secreted HILPDA promote the apolipoprotein uptake by nerve cells

1) HEK293T cells were cultured with DMEM medium in a 10cm cell culture dish.
2) The above cells separately were:
   transfected to express APOE2-3xHA plasmid,
   transfected to express APOE3-3xHA plasmid,
   transfected to express APOE4-3xHA plasmid,
   co-transfected with plasmid expressing HILPDA-Flag and APOE2-3xHA plasmid,
   co-transfected with plasmid expressing HILPDA-Flag and APOE3-3xHA plasmid, and
   co-transfected with plasmid expressing HILPDA-Flag and APOE4-3xHA plasmid.
3) After 12 hours, the old medium in 1) was replaced with fresh DMEM cell culture medium.
4) After further culturing for 48 hours, the media of the cultured cells in 3) were collected, respectively, and these media were used to directly culture recipient neuronal cells, wherein the neuronal cells were obtained by induction and differentiation of human embryonic stem cells (WA09-H9 Wincell Research Institute) through STEMdiff Neural (STEMCELL Technologits) differentiation system.
5) After culturing for 24 hours, anti-Actin (Cat. No.: Proteintech, 66009-1-Ig) and anti-HA antibody (Cat. No.: ab9110, abcam; used to detect three fusion proteins expressed by different alleles APOE2-3xHA, APOE3-3xHA or APOE4-3xHA) were used to perform western blotting.
6) The contents of APOE-3xHA were observed (as shown in Figure 13).

Figure 13 shows the uptake of APOE by recipient cells in the cell culture medium in the presence and absence of HILPDA.

The experimental results showed that: compared with the recipient neuron cells cultured with the culture medium for culturing HEK293T cells expressing APOE2-3xHA, APOE3-3xHA or APOE4-3xHA protein, the recipient neuronal cells cultured with the culture medium for culturing HEK293T cells co-expressing HILPDA-Flag and APOE2-3xHA, and APOE3-3xHA or APOE4-3xHA protein had higher levels of APOE, indicating that the recipient cells absorbed more apolipoprotein APOE2, APOE3 or APOE4 in the presence of HILPDA (indicated by anti-HA bands), and also suggesting that HILPDA promoted the clearance of apolipoproteins and lipoproteins formed therefrom in the nervous system.

### Example 11: Secreted HILPDA promote the apolipoprotein uptake by brain cells

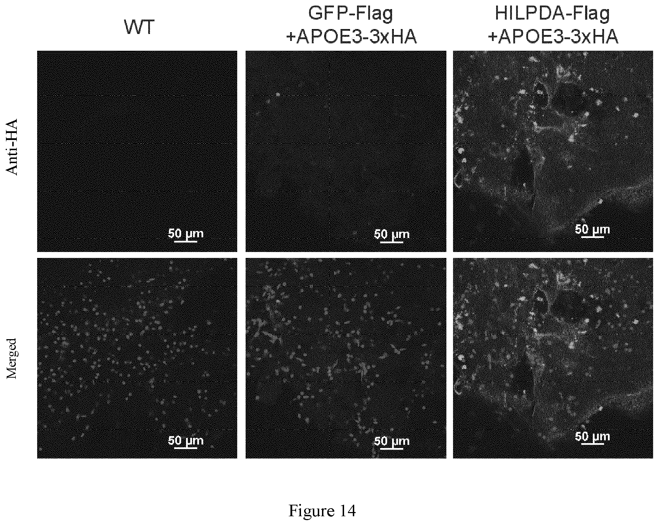

1) HEK293T cells were cultured with DMEM medium in a 10cm cell culture dish.
2) The above cells were separately co-transfected with:
   plasmid expressing GFP-FLAG and APOE3-3xHA plasmid, and
   plasmid expressing HILPDA-Flag and APOE3-3xHA plasmid,
3) After 12 hours, the old medium in 1) was replaced with fresh DMEM cell culture medium.
4) After further culturing for 48 hours, the media of the cultured cells in 3) were collected, respectively, and these media were used to directly culture mouse brain slices, wherein the brain slices were obtained by slicing fresh mouse brains to reach a thickness of 150 µm by a vibrating microtome. The slices were cultured in DMEM medium.
5) After culturing for 48 hours, immunofluorescent staining experiments were carried out with anti-HA antibody (Cat. No.: ab9110, abcam; for detecting APOE3-3xHA fusion protein).
6) The contents of APOE3-3xHA were observed (as shown in Figure 14).

Figure 14 shows the uptake of APOE by brain slices in cell culture medium in the presence and absence of HILPDA.

The experimental results showed that: compared with the brain slices cultured in the culture medium for culturing HEK293T cells co-expressing GFP-Flag and APOE3-3xHA protein, the brain slices cultured in the culture medium for culture HEK293T cells co-expressing HILPDA-Flag and APOE3-3xHA protein showed more and stronger fluorescent signals, indicating that the recipient cells absorbed more apolipoprotein APOE3 in the presence of HILPDA (indicted by anti-HA antibody group), and also suggesting that HILPDA promoted the clearance of apolipoproteins and lipoproteins formed therefrom in the nervous system.

Without being limited to any theory, the present inventors speculate that the mechanism of action of HILPDA is to promote the absorption of lipoproteins in blood or cerebrospinal fluid by human cells through a series of extracellular and intracellular signal transduction pathways, thereby exhibiting prophylactic or therapeutic effect on hyperlipidemia and atherosclerosis and associated co-morbidities, as well as neurodegenerative diseases affected by lipid metabolism. Since the naturally occurring small protein or polypeptide is derived from the human body itself, it should have low toxicity when administered to patients and thus is suitable for clinical transformation and promotion. Therefore, HILPDA will be a polypeptide drug with medical and commercial value.

Although specific models for carrying out the present invention have been described in detail, those skilled in the art will understand that based on all the demonstrations that have been disclosed, various modifications and substitutions can be made to those details, and these changes are all within the scope of the invention. The full scope of the present invention is given by the appended claims and any equivalents thereof.

## Claims

1. Use of a hypoxia-inducible lipid droplet-associated protein or a truncated active fragment thereof in the preparation of a medicament for the treatment or prevention of hyperlipidemia, a hyperlipidemia complication or a neurodegenerative disease, or in the preparation of a medicament for reducing the level of apolipoprotein, the level of low-density lipoprotein or the level of very-low-density lipoprotein in blood or cerebrospinal fluid.

2. The use according to claim 1, wherein the hypoxia-inducible lipid droplet-associated protein has an amino acid sequence as set forth in any one of SEQ ID NOs: 1 to 12.

3. The use according to claim 1, wherein the truncated active fragment of the hypoxia-inducible lipid droplet-associated protein has an amino acid sequence as set forth in any one of SEQ ID NOs: 13 to 15.

4. The use according to any one of claims 1 to 3, wherein the hyperlipidemia is a type III hyperlipidemia.

5. The use according to any one of claims 1 to 3, wherein the hyperlipidemia complication is selected from the group consisting of atherosclerosis, coronary heart disease, hypertension, hyperglycemia and hyperuricemia.

6. The use according to any one of claims 1 to 3, wherein the neurodegenerative disease is selected from the group consisting of multiple sclerosis, Alzheimer's disease and Parkinson's disease.

7. The use according to any one of claims 1 to 3, wherein the apolipoprotein is one or more selected from the group consisting of human APOE2, APOE3 and APOE4.

8. A hypoxia-inducible lipid droplet-associated protein or a truncated active fragment thereof, which is used for treating or preventing a hyperlipidemia, hyperlipidemia complication or neurodegenerative disease, or for reducing the level of apolipoprotein, the level of low-density lipoprotein, or the level of very-low-density lipoprotein in blood or cerebrospinal fluid.

9. The hypoxia-inducible lipid droplet-associated protein or a truncated active fragment thereof according to claim 8, wherein the hypoxia-inducible lipid droplet-associated protein has an amino acid sequence as set forth in any one of SEQ ID NOs: 1 to 12.

10. The hypoxia-inducible lipid droplet-associated protein or a truncated active fragment thereof according to claim 8, wherein the truncated active fragment of the hypoxia-inducible lipid droplet-associated protein has an amino acid sequence as set forth in any one of SEQ ID NOs: 13 to 15.

11. The hypoxia-inducible lipid droplet-associated protein or a truncated active fragment thereof according to any one of claims 8 to 10, wherein the hyperlipidemia is a type III hyperlipidemia.

12. The hypoxia-inducible lipid droplet-associated protein or a truncated active fragment thereof according to any one of claims 8 to 10, wherein the hyperlipidemia complication is selected from the group consisting of atherosclerosis, coronary heart disease, hypertension, hyperglycemia and hyperuricemia.

13. The hypoxia-inducible lipid droplet-associated protein or a truncated active fragment thereof according to any one of claims 8 to 10, wherein the neurodegenerative disease is selected from the group consisting of multiple sclerosis, Alzheimer's syndrome and Parkinson's disease.

14. The hypoxia-inducible lipid droplet-associated protein or a truncated active fragment thereof according to any one of claims 8 to 10, wherein the apolipoprotein is one or more selected from the group consisting of human APOE2, APOE3 and APOE4.

15. A method of treating or preventing hyperlipidemia, a hyperlipidemia complication or a neurodegenerative disease, or a method of reducing the level of apolipoprotein, the level of low-density lipoprotein or the level of very-low-density lipoprotein in blood or cerebrospinal fluid, comprising a step of administering to a subject in need thereof an effective amount of a hypoxia-inducible lipid droplet-associated protein or a truncated active fragment thereof.

16. The method according to claim 15, wherein the hypoxia-inducible lipid droplet-associated protein has an amino acid sequence as set forth in any one of SEQ ID NOs: 1 to 12.

17. The method according to claim 15, wherein the truncated active fragment of the hypoxia-inducible lipid droplet-associated protein has an amino acid sequence as set forth in any one of SEQ ID NOs: 13 to 15.

18. The method according to any one of claims 15 to 17, wherein the hyperlipidemia is a type III hyperlipidemia.

19. The method according to any one of claims 15 to 17, wherein the hyperlipidemia complication is selected from the group consisting of atherosclerosis, coronary heart disease, hypertension, hyperglycemia and hyperuricemia.

20. The method according to any one of claims 15 to 17, wherein the neurodegenerative disease is selected from the group consisting of multiple sclerosis, Alzheimer's disease and Parkinson's disease.

21. The method according to any one of claims 15 to 17, wherein the apolipoprotein is one or more selected from the group consisting of human APOE2, APOE3 and APOE4.

22. A truncated active fragment of hypoxia-inducible lipid droplet-associated protein, which has an amino acid sequence as set forth in any one of SEQ ID NOs: 13 to 15.

23. A pharmaceutical composition, which comprises an effective amount of the truncated active fragment of hypoxia-inducible lipid droplet-associated protein according to claim 22, and one or more pharmaceutically acceptable excipients;
optionally, the pharmaceutical composition further comprises one or more blood lipid-lowering drugs such as statins.

24. A combination pharmaceutical product, which comprises a first product and a second product that are separately packaged,
wherein,
the first product comprises the pharmaceutical composition according to claim 23;
the second product comprises one or more blood lipid-lowering drugs such as statins; optionally, a product manual is also comprised.
